# EUROPEAN PATENT APPLICATION

(11) **EP 0 959 133 A1**
(43) Date of publication of application: **24.11.1999**
(21) Application number: 98201666.9
(22) Date of filing: 22.05.1998
(51) Int. Cl.: C12N 15/63, C12N 15/67, C12N 15/90, A01H 5/00

(54) **A process for inhibiting expression of genes**

(71) Applicant: CENTRUM VOOR PLANTENVEREDELINGS- EN REPRODUKTIEONDERZOEK (CPRO-DLO), 6708 PB Wageningen (NL)
(72) Inventor: Nap, Jan-Peter Hendrik, NL 6708 PB Wageningen (NL); Mlynarova, Ludmila, NL 6708 PB Wageningen (NL); Stiekema, Willem Johannes, NL 6708 PB Wageningen (NL)
(74) Representative: De Hoop, Eric

(57) **Abstract**

For inhibiting the expression of a gene or a group of homologous genes present in the genome of an organism, a nucleotide sequence is introduced in the organism, which nucleotide sequence has the configuration A - interrupt - B. A and B, which may be the same or different, are selected from the group consisting of the gene to be inhibited or one of the homologous genes, any part thereof comprising at least 30 bp, and any homologue thereof. "Interrupt" is a random nucleotide sequence which may comprise any nucleotide sequence, e.g. a gene or a selection marker. The transcription directions of A and B may be the same or opposite.

## Description

This invention relates to a process for inhibiting expression of a gene or a group of homologous genes present in the genome of an organism.

Whereas it is known that gene expression can be affected by antisense strategies (see Bourque, 1995), or reintroduction of the sequence of interest (sense strategies, see Kooter & Mol, 1993), the efficiency of these approaches is low. There is a need for a method that can reduce gene expression more efficiently and reliably.

The present invention provides a process for inhibiting expression of a gene or a group of homologous genes present in the genome of an organism, the process comprising introducing in said organism a nucleotide sequence having the following configuration:
A - interrupt - B
wherein A and B, which may be the same or different, are selected from the group consisting of said gene or one of said homologous genes, any part thereof comprising at least 30 bp, and any homologue thereof,
"interrupt" is a random nucleotide sequence, and the transcription directions of A and B may be the same or opposite.

The present invention also relates to nucleotide sequences useful in the method, and to vectors comprising said nucleotide sequences.

A key advantage of the present invention is the efficiency by which inhibition of gene expression in transgenic organisms is obtained. The method of the present invention affects gene expression more efficiently and reliably when compared to known methods of affecting gene expression. With respect to this advantage of the present invention, the efficiency of the method of the present invention obviates the need for generating and analyzing large numbers of organisms for the intended reduction in gene expression.

### Definitions

The term "nucleotide sequence" in relation to the present invention includes DNA and RNA. Preferably it means DNA, more preferably DNA prepared by use of recombinant DNA techniques.

The term "... any homologue thereof" in relation to the nucleotide sequence of the present invention includes any substitution of, variation of, modification of, replacement of, deletion of one or more nucleotides (nucleic acids) from or addition to the respective gene. In particular, the term "homologue" covers homology with respect to similarity of structure and/or similarity of function. The above terms also include allelic variations of the sequences. For example, a DNA sequence of 2 kb may be homologous in a region of 100 bp and reduce gene expression in accordance with the present invention. Homologous in this case means "identical" or "similar" for at least 50%, preferably 70%, or more. Over the entire sequence the homology may be low. In general a homologue is here defined as "any nucleotide sequence having a similarity for at least 50%, preferably 70%, or more, over a sequence of 60 bp or more".

The term "vector" includes an expression vector and a transformation vector. The term "expression vector" comprises a construct capable of expression in all in vivo or in vitro expression systems. The term "transformation vector" comprises a construct capable of being transferred from one organism to another.

The term "promoter" comprises all possible enhancer sequences and is used in the present invention as nucleotide sequence that binds suitable transcription factors and RNA polymerase, resulting in RNA in the organism or any part thereof. The promoter therefore includes any conserved DNA regions, and DNA regions that affect the level of RNA formation, such as leader sequences, and any DNA regions that give rise to induction of RNA synthesis as result of stress, chemicals, light, temperature, cell, tissue or organ specificity or any other inducing agent, as well as any fragments, variants or homologues of the DNA regions providing the resultant DNA region gives rise to RNA production in an organism or any part thereof.

The term "gene" includes normally a promoter sequence, a coding sequence, optionally introns and a terminator sequence.

The nucleotide sequence to be introduced in the present process comprises two genes or parts thereof, A and B, interrupted by a random nucleotide sequence. Said configuration is hereinafter indicated as interrupted repeat configuration. The transcription directions of A and B may be the same or opposite. In a preferred embodiment the transcription directions of A and B are opposite.

The interrupting random sequence may comprise any nucleotide sequence, e.g. a gene, a selection marker, a matrix-associated region, etc. The length of the random sequence is not critical. It appeared not to be possible to clone or maintain inverted repeats without an interrupting sequence. Apparently, such inverted repeats are not stable. Any length of the random sequence may be suitable providing it contributes to a stable maintenance of the repeat configuration; preferably the random sequence will be less than 10 kb. Preferably the interrupting nucleotide sequence will be a selectable marker gene.

In a preferred embodiment the two genes or parts thereof, A and B, comprise a promoter attached to the coding sequence of a gene or both genes. Any promoter can be used, either the natural promoter of the coding sequence or a foreign promoter, which is any promoter from any gene from any other organism. As foreign promoters preferably highly effective promoters are used, e.g. a dCaMV 35 promoter in case of plants.

The two genes or parts thereof, A and B, may furthermore include any transcription enhancement or translation enhancement regions, as well as a transcriptional termination region from either the natural gene or from any other gene.

The gene to be inhibited can be either natural or foreign to the organism. The gene does not necessarily code for a protein or an enzyme, but if it does, the encoded protein or enzyme may be non-natural to the receiving organism.

The present process can be used to inhibit the expression of genes in the genome of any organism from any taxonomical origin that could receive an interrupted repeat configuration according to the present invention. The present invention therefore includes mammals, including human beings, plants, micro-organisms, fungi and other, or any part thereof. Preferably, the term "plant" as used in this invention includes any suitable angiosperm, gymnosperm, monocotyledonous or dicotyledonous species or any part thereof.

Although the examples illustrating the invention relate to the inhibition of gene expression in plants, it is contemplated that the invention can be practised in other organisms too.

When the interrupted repeat configuration has been introduced in an organism, a tranegenic organism has been made. Preferably the interrupted repeat configuration is incorporated in or stably added to the genome of the transgenic organism.

Accordingly, the invention also relates to a transgenic organism or any part thereof obtained by the process of the invention or comprising the nucleotide sequence used in the process. The term "or any part thereof" includes all cells, tissues and organs, both within the organism during its complete life cycle, and all cells, tissues and organs per se, as well as derivatives of all cells, tissues and organs.

The process of the invention can be used as a tool in gene function analysis in case of gene redundancy. A group of sufficiently similar or homologous target genes will be affected simultaneously with the introduction of one of these genes or homologues in an interrupted repeat configuration of the invention. As a result a group of gene products that are redundant in the organism are reduced or eliminated. The possibility of reducing down a group of sufficiently homologous target genes with a gene in an interrupted repeat configuration will complement prior art methods such as mutation and transposon tagging in gene function search, especially in case of redundant genes.

A further aspect of the present invention relates to elucidating the function of genes with hitherto unknown roles. The present invention allows the function of a group of homologous or partially homologous genes which may be redundant or overlapping in biological function, to be evaluated. This particular aspect of the present invention is advantageous over the prior art methods which, for example, rely on the elimination or mutation of a single gene.

The basic principle in the construction of a genetically modified or transgenic organism is to insert genetic information in or to add to the genome so as to obtain stable maintenance of the introduced genetic material. The skilled person will appreciate that transformation of a particular organism can be carried out using techniques well known in the art. The techniques for inserting genetic material depend on the particular organism and are known to experts in the field. For plants, this could involve the use of the Agrobacterium transformation methods, or all other methods suitable for introducing genes into plants.

The present invention will now be illustrated by the following examples, which should not be construed as in any way limiting the invention. The examples particularly illustrate the process of the invention in which the transcription directions of A and B in the interrupted repeat configuration are opposite. Said configuration is indicated as interrupted inverted repeat, abbreviated as INTIR. In the examples the result of the process of the invention is also referred to as gene silencing.

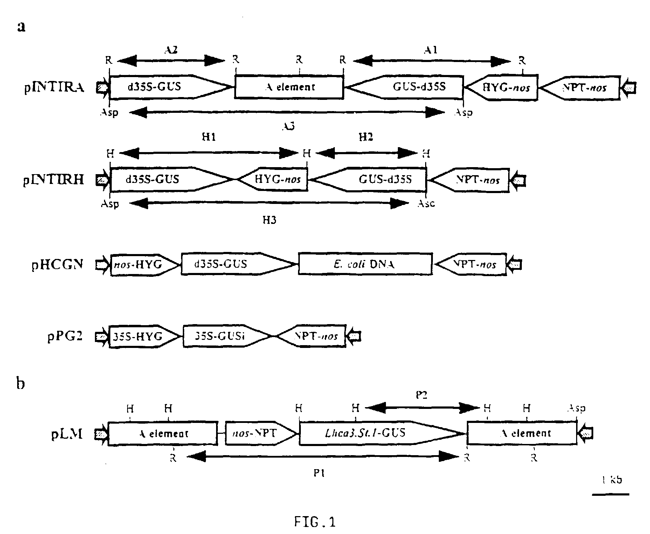

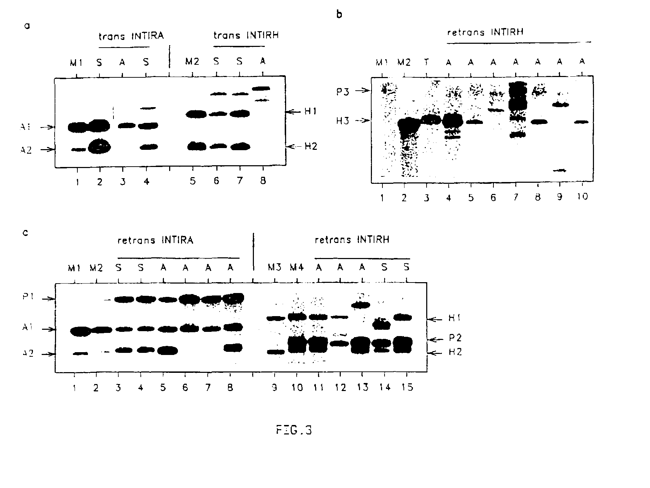

The examples refer to the following Figures in which
**Figure 1** shows T-DNA configurations used in these examples.
   **a**, Plant vectors used for transformation of the wild type (WT) and ANLGA-13 tobacco. The direction of transcription of the respective genes is given by the shape of the box. The pHCGN vector originates from our attempts to clone the GUS gene as a direct inverted repeat. The plasmid contains a fortuitously cloned piece of unknown *E*. *coli* DNA. The striped arrows indicate the borders of the *Agrobacterium* T-DNA. The restriction enzyme fragments used for DNA blot analysis (see Fig. 3) are indicated with a doubleheaded arrow and the fragment identification code A or H.
   **b**, pLM T-DNA; tobacco ANLGA-13 used for regeneration and retransformation carries a single copy of the pLM T-DNA. The restriction enzyme fragments used for DNA blot analysis (see Fig. 3) are indicated with a doubleheaded arrow and the fragment identification code P.
      Abbreviations: 35S, single CaMV 35S promoter; d35S, doubled CaMV 35S promoter; GUS, β-glucuronidase gene; GUSi, GUS with potato intron; HYG, hygromycin phosphotransferase gene; *Lhca3.St.1,* promoter from the potato (*Solanum tuberosum*) gene encoding a protein of the Photosystem I light harvesting complex; nos, nopaline synthase promoter; NPT, neomycin phosphotransferase gene; restriction enzymes: Asc, *Asc*I; Asp, *Asp*718I; H, *Hin*dIII; R, *Eco*RI.

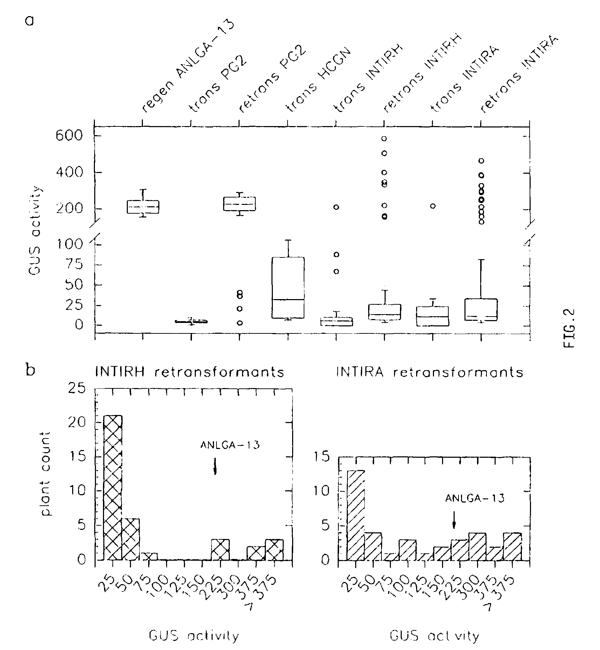

**Figure 2** shows distributions of GUS activity in control, transformed and retransformed plant populations.
   **a**, Box plots of the GUS activity in the respective plant populations.
      Each horizontal line represents the 10th, 25th, 50th (median), 75th and 90th percentiles; populations are indicated on the top; GUS activity is given in pmol methylumbelliferon per minute per microgram of soluble protein. The open circles are the GUS activities of the individual plants not taken into account by making the box plot. These are the silenced plants in case of pPG2 retransformation; and the non-silenced plants in case of pINTIRA and pINTIRH transformation of wild-type tobacco (activity > 60) and pINTIRA and pINTIRH retransformation of ANLGA-13 tobacco (activity > 108).
   **b**, Histogram of data from the pINTIRH and pINTIRA retransformation of tobacco ANLGA-13. The activity of the ANLGA-13 parent is given by an arrow.
**Figure 3** shows DNA blot analysis of INTIR-transformed and - retransformed plants probed with a GUS probe.
   **a**, Analysis of *Eco*RI-digested genomic plant DNA isolated from INTIRH and INTIRA transformants after transformation of wild-type tobacco. On top, the GUS activity is indicated with S for a silenced and with A for an active transformant. Both active transformants (lanes 3 and 8) have not the expected pattern of bands. The expected fragments A1, A2, H1 and H2 (see Fig. 1) are indicated by arrows. M1, M2 are control lanes with the expected fragments added to salmon sperm DNA.
   **b**, *Asp*718I+*Asc*I-digested genomic plant DNA isolated from INTIRH retransformants after transformation of tobacco ANLGA-13. The lane labeled T on top is an INTIRH transformant, all lanes labeled A on top contain DNA from plant line that have GUS activity. The lines in lanes 5, 8 and 10 received intact INTIRH DNA. The expected fragments H3 and P3 are indicated by arrowheads. M1 contains ANGLA-13 DNA; fragment P3 represents the whole T-DNA of pLM plus a fragment of unknown length of tobacco genomic DNA. M2 contains the expected fragment H3 added to salmon sperm DNA.
   **c**, Analysis of genomic plant DNA from INTIRA and INTIRN retransformants obtained after transformation of tobacco line ANLGA-13, showing the stable maintenance of the INTIR configuration in plants. The DNA was digested with *Eco*RI (INTIRA) or *Hind*III (INTIRH). On top, the GUS activity is indicated with S for a silenced and with A for an active retransformant. The expected INTIR-derived fragments A1, A2, H1 and H2 (see Fig. 1) are indicated by arrows, as well the expected fragments P1 and P2 from the parental LM T-DNA (see Fig. 1). M1-4 are control lanes with the expected INTIR fragments added to salmon sperm DNA (M1, M3) and to ANLGA-13 DNA (M2, M4).

### Examples.

The gene used in the examples consists of the doubled cauliflower promoter 35S promoter (dCaMV 35S) driving the bacterial β-glucuronidase (GUS) reporter gene. The two INTIR constructs used for plant transformation are given in Fig. 1a. The interrupting sequences used were the *nos* promoter-driven hygromycin resistance gene (pINTIRH) and the non-coding chicken lyzozyme A element (pINTIRA).

The dCaMV 35S promoter-GUS gene by itself (Fig. 1a: pHCGN) yields average GUS expression levels upon transformation (Fig. 2a: trans HCGN), whereas the single CaMV promoter-GUS fusion (Fig 1a: pPG2) results in overall low GUS activity levels (Fig 2a: trans PG2). Transformation of wild-type tobacco with either pINTIRH and pINTIRA resulted primarily in transgenic plants with GUS activities that were low or below the level of detection (Fig. 2a: trans INTIRH and trans INTIRA). Gene silencing is therefore operational within INTIR loci, irrespective of their place of integration. DNA blot analysis showed the proper integration and stable maintenance of the INTIR configuration in plants (Fig. 3a, all lanes labeled S). Few plants, 3 out of 42 in case of pINTIRH and 1 out of 36 in case of pINTIRA, showed the level of expression of the dCAMV 35S promoter-GUS fusion (Fig. 2a). Subsequent DNA blot analyses showed that in these cases the T-DNA transfer had been incomplete (examples in Fig. 3a, lanes 3 and 8). As DNA transfer or integration stopped within the repeat-interrupting sequence, these plants effectively received a single copy of the dCAMV 35S-GUS transgene, resulting in GUS activity.

To determine if the INTIR constructs were capable of silencing endogenous genes, tobacco line ANLGA-13 carrying a single GUS gene was chosen for retransformation experiments. This line is homozygous for a single copy of the pLM T-DNA (Fig. 1b). The *Lhca3.St.1* promoter-GUS gene expression in this line was stable and fully additive (Nap et al., 1997) over at least four generations of selfing. To further substantiate the intrinsic stability of GUS activity in this tobacco line, 47 regenerants were obtained by mock transformation. GUS activity in these regenerants was similar to the activity in the parent plant (Fig. 2a: regen ANLGA-13). The homozygous GUS gene in tobacco line ANLGA-13 therefore behaves as a fully stable endogenous gene and is a suitable target for silencing by retransformation assays.

In the retransformants of ANLGA-13 obtained after retransformation with vector pPG2 (Fig. 1a), the expected 10% (4 out of 40) of plants showed a strong reduction of GUS activity (Fig. 2a: retrans PG2; Table 1). In contrast, retransformation with either pINTIRA or pINTIRH resulted in much more gene silencing. The distribution of GUS activities in the populations of ANLGA-13/INTIRH and ANLGA-13/INTIRA retransformed plants is given in Fig. 2 (retrans INTIRH and retrans INTIRA). Out of 36 ANLGA-13/INTIRH retransformants, 29 (81%) showed considerable reductions in GUS activity; out of 38 ANLGA-13/INTIRA retransformants, 22 (58%) plants (Table 1). GUS activity ranged from 45 to almost 0 % of the ANLGA-13 parental activity; on the average 6-7% residual GUS activity was retained (Table 1). The GUS activity in the 21 active retransformants may define the requirements for efficient INTIR-mediated gene silencing. Activity could be due to several reasons, such as a complete but ineffective INTIR configuration or to an incomplete INTIR integration (as in case of the transformation assay shown above). DNA blot analysis of the active retransformants showed both cases to occur (Fig. 3b, lanes 4-10).

**Table 1**

| **Retransformation with INTIR DNA results in efficient gene silencing** | | | | | | |
|---|---|---|---|---|---|---|
| DNA | number of plants | | mean GUS activity* in plants | | | |
| | total^{†} | silenced^{††} | % silenced | active | silenced | % of parent |
| none | 47 | 0 | 0 % | 217 | n.a. | n.a. |
| PG2 | 40 | 4 | 10 % | 225 | 14.2 | 6.5 % |
| INTIRH | 36 (28) | 29 (25) | 81 % (89 %) | 307 | 13.8 | 6.4 % |
| INTIRA | 38 (34) | 22 (22) | 58 % (65 %) | 245 | 15.8 | 7.2 % |

| | | | | | | |
|---|---|---|---|---|---|---|
| *mean GUS activities are the backtransformed natural logarithmic (i.e. geometric) means. | | | | | | |
| ^{†}Numbers between brackets refer to the number of plants that received at least one intact INTIR configuration. | | | | | | |
| ^{††}Plants are classified 'silenced' if they show less than half the GUS activity of the ANLGA-13 parent (i.e.<108 pmol MU/min.Tg protein); n.a., not applicable. | | | | | | |

In total nine retransformants (3 INTIRH, 6 INTIRA) showed GUS activity, whereas DNA blot and genetic analysis of hygromycin resistance indicated the presence of an intact integration of the INTIR DNA. Fig. 3b (lanes 5, 8 and 10) shows the presence of a single full INTIR-containing DNA fragment for three active INTIRH retransformants; Fig 3c (lanes 5, 8 and 15) shows the presence of the two expected INTIR DNA fragments. This result implies that an INTIR configuration *per se* is not fully sufficient for gene silencing. Whereas the majority of genomic positions is generating the INTIR-mediated gene silencing, few positions may escape the phenomenon, possibly due to a rare position effect.

Twelve retransformants (4 INTIRA, 8 INTIRH) received the dCaMV 35S-GUS transgene due to an incomplete INTIR integration (Fig. 3c, lanes 6, 7, 12, 14) . Four of these twelve (30%, all INTIRH retransformants) were silenced (Fig. 3c, lane 14). Introduction of the dCaMV 35S-GUS transgene alone is apparently not resulting in a high silencing percentage, in contrast to a dCaMV 35S promoter-chalcone synthase construct in *Petunia hybrida* (Que et al., 1997). However, the DNA blot analysis cannot exclude that a small part of the 3' end of the second GUS gene was transferred as well. This may be sufficient for gene silencing (Sijen et al., 1996; English et al.,1996). For assessing the efficacy of INTIR constructs it is reasonable to limit the plant populations to those transformants that received at least one intact INTIR configuration. Analysed this way, the percentage of silencing ranges from 65% for INTIRA to as high as 89% for INTIRH (Table 1).

This high efficiency of gene silencing observed in INTIR-containing plants indicates that the INTIR-configuration by itself is in the majority of cases sufficient to trigger gene silencing. The presence of a transcribed gene (pINTIRH), or a matrix-associated region (pINTIRA), as interruption of the inverted repeated sequence does not interfere with this triggering as long as the intact INTIR is present. This opens up the INTIR approach as relatively simple and hence attractive method to shut down expression of genes by homology. Further experiments will define possible constraints for the interrupting sequence (length, sequence content) as well as for the genes to affect by gene silencing (homology or transcriptional requirements, promoter strength, mRNA stability), equivalent to constraints for antisense (Bourque et al., 1995; Matteucci et al., 1996) and sense suppression (Sijen et al., 1996; Que et al., 1997) approaches. The high efficiency will also uncouple chromosomal position effects from the putative physical instability of an INTIR configuration, as demonstrated for a large IR in mice (Collick et al., 1996) or from the often observed somatic instability of the gene silencing phenomenon (Baulcombe D.C., 1996; Meins et al., 1995). Data from our laboratory indicate that both DNA configuration and gene silencing survive meiosis in tobacco.

Genome sequencing has amply indicated that gene redundancy is the rule rather than an exception, even in the highly compact genome of *Arabidopsis thaliana.* Efficient simultaneous reduction of the expression of a group of redundant genes will allow a first classification of the function of such a gene group, preceding the formal genetic proof of any assigned function(s) by mutation(s). Recently the 'amplicon' system based on viral replication was proposed for consistent gene silencing in plants (Angell et al., 1997). As INTIR transformation is not limited to the host range of a virus, it would seem to allow easier evaluation and study of gene silencing phenomena in plants as well as in organisms other than plants. Recent results in *Drosophila* (Pal-Bhadra et al., 1997) suggest that gene silencing may indeed belong to the basic repertoire of all organisms to regulate their gene expression.

### Methods

**Plant vectors.** Plasmid pPG2 is a pBin19-derivative (Mlynarova et al., 1994) that carries the single CaMV 35S promoter driving the E. *coli* GUS gene with a potato intron (Vancanneyt et al., 1990). The other vectors carry a doubled CaMV 35S promoter driving the GUS gene without intron. This dCaMV 35S-GUS gene as *Sal*I - *Bam*HI fragment from pLM9 (ANGA) (Mlynarova et al., 1995) and the *nos-*HYG gene as *Asp*718I-*Hin*dIII fragment from pPCV720 (Koncz, et al., 1994) were cloned in two orientations in the *Eco*RV site of pBluescript SK+ or KS+ (Stratagene). The A element was isolated from plasmid pUCB1X1 (Mlynarova et al., 1994). Fragments were assembled and cloned in the transformation vector pBinPLUS (Van Engelen et al., 1995) by multipoint ligations using suitable restriction sites, and transformed into *E*. *coli* DH5IF'. After verification, plasmids were introduced in *E*. *coli* S17.1 and conjugated to *Agrobacterium tumefaciens* LBA4404 by biparental mating (Mlynarova et al., 1994).

**Plant transformation**. Tobacco *(Nicotiana tabacum* cv. Petit Havana SR1) leaves were transformed with *A*. *tumefaciens* as previously described (Mlynarova et al., 1994), with selection on 20 µg/ml hygromycin. *In vitro* shoots that rooted in the presence of this antibiotic were transferred to the greenhouse with previously described growth conditions. In the regeneration of ANLGA-13, no hygromycin selection was applied.

**GUS assays.** GUS activity in tobacco leaves was measured by fluorometry using a Titertek Fluoroscan II (Flow Laboratories) as previously described (Mlynarova et al., 1994).

**DNA analysis**. Total DNA was isolated from leaves of six-week-old greenhouse-grown tobacco plants as described (Mlynarova et al., 1994). For DNA blot analysis, 10 µg was digested with the appropriate restriction enzymes, separated on 0.7-1.0 % agarose gels, blotted onto HyBond (Amersham) and hybridized with random-prime labeled probes (Amersham), according to established procedures. As size and hybridization markers, isolated fragments were mixed with either 10 µg sheared salmon sperm DNA or ANGLA-13 DNA prior to gel electrophoresis.

### References

Angell, S.M. & Baulcombe, D.C. Consistent gene silencing in transgenic plants expressing a replicating Potato Virus X RNA. *EMBO J*. **16**, 3675-3684 (1997).

Baulcombe, D.C. RNA as a target and an initiator of post-transcriptional gene silencing in transgenic plants. *Plant Mol. Biol*. **32,** 79-88 (1996).

Bourque, J.E. Antisense strategies for genetic manipulations in plants. *Plant Sci.* **105,** 125-149 (1995).

Collick, A. *et al*. Instability of long inverted repeats within mouse transgenes. *EMBO J*. **15,** 1163-1171 (1996).

English, J.J., Mueller, E. & Baulcombe, D.C. Suppression of virus accumulation in transgenic plants exhibiting silencing of nuclear genes. *Plant Cell* **8,** 179-188 (1996).

Koncz, C. *et al*. Specialized vectors for gene tagging and expression studies. in *Plant Molecular Biology Manual* (eds Gelvin, S.B. & Schilperoort, R.A.) B2: 1-22 (Kluwer, Dordrecht, 1994).

Kooter, J.M. & Mol, J.N.M. Trans-inactivation of gene expression in plants. *Curr. Opin. Biotechn.* **4,** 166-171 (1993). Matteucci, M.D. & Wagner, R.W. In pursuit of antisense. *Nature* **384**, 20-22. (1996).

Meins Jr., F. & Kunz, C. Gene silencing in transgenic plants: a heuristic autoregulatory model. *Curr. Top. Microbiol. Immun.* **197**, 105-120 (1995).

Mlynarova, L. *et al*. Reduced position effect in mature transgenic plants conferred by the chicken lysozyme matrix-associated region. *Plant Cell* **6**, 417-426 (1994).

Mlynarova, L., Jansen, R.C., Conner, A.J., Stiekema, W.J. & Nap, J.P. The MAR-mediated reduction in position effect can be uncoupled from copy number-dependent expression in transgenic plants. *Plant Cell* **7**, 599-609 (1995).

Nap, J.P., Conner, A.J., Mlynarova, L., Stiekema, W.J. & Jansen, R.C. Dissection of a synthesized quantitative trait to characterize transgene interactions. *Genetics* **147**, 315-320 (1997).

Pal-Bhadra, M., Bhadra, U. & Birchler, J.A. Cosuppression in Drosophila: gene silencing of *alcohol dehydrogenase* by *white adh* transgenes is *polycomb* dependent. *Cell* **90**, 479-490 (1997).

Que, Q.D., Wang, H.Y., English, J.J. & Jorgensen, R.A. The frequency and degree of cosuppression by sense chalcone synthase transgenes are dependent on transgene promoter strength and are reduced by premature nonsense codons in the transgene coding sequence. *Plant Cell* **9**, 1357-1368 (1997).

Sijen, T., Wellink, J., Hiriart, J.B. & van Kammen, A. RNA-mediated virus resistance: role of repeated transgenes and delineation of targeted regions. *Plant Cell* **8**, 2277-2294 (1996).

Vancanneyt, G., Schmidt, R., O'Connor Sanchez, A., Willmitzer, L. & Rocha Sosa, M. Construction of an intron-containing marker gene: Splicing of the intron in transgenic plants and its use in monitoring early events in *Agrobacterium* mediated plant transformation. *Mol.* G*en. Genet*. **220,** 245-250 (1990).

Van Engelen, F.A. *et al*. pBINPLUS: An improved plant transformation vector based on pBIN19. *Transgenic Res*. **4**, 288-290 (1995).

## Claims

1. A process for inhibiting expression of a gene or a group of homologous genes present in the genome of an organism, the process comprising introducing in said organism a nucleotide sequence having the following configuration:
A - interrupt - B
wherein A and B, which may be the same or different, are selected from the group consisting of said gene or one of said homologous genes, any part thereof comprising at least 30 bp, and any homologue thereof,
"interrupt" is a random nucleotide sequence, and
the transcription directions of A and B may be the same or opposite.

2. A process according to claim 1, wherein the transcription directions of A and B are opposite.

3. A process according to claim 1 or 2, wherein A and/or B comprise a foreign promoter attached to the coding sequence of a gene.

4. A process according to claims 1 to 3, wherein the random nucleotide sequence has a length of up to 10kb.

5. A process according to claims 1-4, wherein the organism is a plant.

6. A nucleotide sequence having the following configuration:
A - interrupt - B
wherein A and B, which may be the same or different, are selected from the group consisting of a gene, any part thereof comprising at least 30 bp, and any homologue thereof,
"interrupt" is a random nucleotide sequence, and
the transcription directions of A and B may be the same or opposite.

7. A nucleotide sequence according to claim 6, wherein the transcription directions of A and B are opposite.

8. A nucleotide sequence according to claim 6 or 7, wherein A and/or B comprise a foreign promoter attached to the coding sequence of a gene.

9. A nucleotide sequence according to claims 6 to 8, wherein the random nucleotide sequence has a length of up to 10 kb.

10. A vector comprising a nucleotide sequence of any of claims 6-9.

11. A transgenic organism or any part thereof obtained by the process of claims 1-5, or comprising the nucleotide sequence of claims 6-9.
